# EUROPÄISCHE PATENTSCHRIFT

(11) **EP 2 895 125 B1**
(45) Veröffentlichungstag und Bekanntmachung des Hinweises auf die Patenterteilung: **09.01.2019**
(21) Anmeldenummer: 13720226.3
(22) Anmeldetag: 19.03.2013
(51) Int. Cl.: A61F 13/02, A61F 13/00

(54) **KLEBEVERBAND**
ADHESIVE DRESSING
PANSEMENT AUTOCOLLANT

(30) Priorität: 26.06.2012 DE 102012105554
(43) Veröffentlichungstag der Anmeldung: 22.07.2015
(73) Patentinhaber: Fögeling-Oomen, Monika, 41539 Dormagen (DE)
(72) Erfinder: Fögeling-Oomen, Monika, 41539 Dormagen (DE)
(74) Vertreter: Cohausz Hannig Borkowski Wißgott
(86) Internationale Anmeldenummer: PCT/EP2013/000823
(87) Internationale Veröffentlichungsnummer: WO 2014/000835

(56) Entgegenhaltungen:
- WO-A1-2011/008360
- DE-A1- 1 963 375
- DE-U1- 9 302 166
- DE-U1-202009 009 404
- US-A- 5 060 662
- US-A- 6 093 160
- US-A1- 2005 107 732
- US-A1- 2007 161 937
- US-A1- 2010 010 458

## Beschreibung

Die vorliegende Erfindung betrifft einen Klebeverband zum Schutz und zur Therapierung offener Wunden, umfassend eine flexible Trägerschicht, die eine die Wunde im bestimmungsgemäßen Zustand freilegende Aussparung definiert, eine an der Unterseite der Trägerschicht vorgesehene selbstklebende Haftschicht, die im bestimmungsgemäßen Zustand zur Fixierung des Klebeverbands dient, eine auswärts von der Haftschicht vorstehende und die Aussparung rahmenartig umgebende Abstandseinrichtung, die derart flexibel ausgebildet ist, dass sie sich unter Einwirkung äußerer Kräfte verformt, und eine die Abstandseinrichtung zumindest oberseitig abdeckende, gitterartig ausgebildete Abdeckschicht aus einem flexiblen Material.

Klebeverbände sind im Stand der Technik in unterschiedlichsten Ausgestaltungen bekannt. Sie dienen dazu, offene Wunden vor Umwelteinflüssen zu schützen. Insbesondere soll ein Eindringen von Fremdkörpern oder Krankheitserregern verhindert werden. Darüber hinaus sollten Klebeverbände zur Sekretaufnahme geeignet sein, um auf diese Weise einem unerwünschten Aufweichen des Gewebes vorzubeugen und den Abtransport heilungshemmender Stoffe zu gewährleisten.

Die DE-U-20 2009 009 404 offenbart beispielsweise einen Klebeverband der eingangs genannten Art, der eine Trägerschicht mit einer mittig vorgesehenen Aussparung aufweist. An der Unterseite der Trägerschicht ist eine selbstklebende Haftschicht angeordnet, und an der Oberseite der Trägerschicht ist ein auswärts von dieser vorstehendes, haubenartig und formstabil ausgebildetes Element vorgesehen, das beispielsweise in Form eines formstabilen Kunststoffgitters ausgebildet ist. Dank der in der Trägerschicht vorgesehenen Aussparung liegt die zu schützende Wunde im bestimmungsgemäß angeordneten Zustand des Klebeverbandes frei, so dass Luft an diese gelangen kann, wodurch der Heilungsprozess beschleunigt wird. Durch die haubenartige Ausbildung des formstabilen Elementes wird ein Abstand zwischen der Trägerschicht und der Oberseite des formstabilen Elementes geschaffen, der derart groß ist, dass selbst bei Austreten von Wundsekret Verklebungen oder Verwachsungen der Wunde mit dem Element ausgeschlossen werden können. Ein Nachteil des in der DE-U-20 2009 009 404 offenbarten Klebeverbandes besteht allerdings darin, dass dieser nur bedingt im veterinärmedizinischen Bereich einsetzbar ist. Die meisten Tiere empfinden insbesondere das auswärts abstehende formstabile Element als störenden Fremdkörper und neigen zu dem Versuch, den Klebeverband zu entfernen, indem sie sich über den Boden wälzen oder an Fremdobjekten entlangstreifen. Ein ähnlicher Klebeverband der eingangs genannten Art ist aus der Schrift US 5,060,662 A bekannt. Hier ist die Abdeckschicht als luftdurchlässige Gaze ausgebildet, um eine hohe Biegsamkeit und Flexibilität zu gewährleisten. Gleichwohl sieht dieser Klebeverband vor, die Gaze durch eine mit ihr verbundene Schutzschicht abzudecken, die weitgehend starr ist, so dass die Abdeckschicht und infolgedessen der Klebeverband an Flexibilität verliert. Des Weiteren beschreibt die US 5,060,662 A keine spezielle Trägerschicht. Vielmehr ist hier die Unterseite der Abstandseinrichtung mit der Haftschicht versehen, die direkt auf die Haut geklebt wird. Die Haftschicht ist von einer Folie bedeckt, die vor dem bestimmungsgemäßen Gebrauch abzuziehen ist.

Ausgehend von dem Stand der Technik ist es eine Aufgabe der vorliegenden Erfindung, einen Klebeverband der eingangs genannten Art zu schaffen, der sowohl in der Humanmedizin als auch im Bereich der Veterinärmedizin einsetzbar ist und eine hohe Flexibilität gewährleistet.

Zur Lösung dieser Aufgabe schafft die vorliegende Erfindung einen Klebeverband der eingangs genannten Art, bei dem die flexible Trägerschicht allseitig unter der Abstandseinrichtung vorsteht. Dies gewährleistet eine verbesserte, großflächige Haftung des Klebeverbands auf der Haut bei gleichzeitig hoher Biegsamkeit und Flexibilität. Ferner führt die flexible Ausbildung der Abstandseinrichtung dazu, dass von außen auf diese einwirkende Kräfte automatisch durch Verformung absorbiert oder zumindest umgelenkt werden. Entsprechend werden auf die Abstandseinrichtung einwirkende Kräfte nur reduziert an die Trägerschicht und an die Haftschicht übertragen, wodurch ein unerwünschtes Abreißen des Klebeverbandes im Regelfall sicher verhindert wird. Zur flexiblen Ausbildung der Abstandseinrichtung ist diese aus einem flexiblen Material hergestellt, insbesondere aus einem Schaumstoff, Vlies oder dergleichen.

Gemäß einer Ausgestaltung der vorliegenden Erfindung ist die Trägerschicht aus einem stretchfähigen Material hergestellt, insbesondere aus einem stretchfähigen Gewebe oder Kunststoffmaterial, das der Trägerschicht eine Querelastizität verleiht, wie beispielsweise Polyestervlies oder dergleichen. Eine solche Stretchfähigkeit unterstützt eine sichere Fixierung des Klebeverbands auch unter der Einwirkung äußerer Kräfte, da ein Teil der Kräfte durch entsprechende Ausdehnung des Trägerschichtmaterials absorbiert wird. Die Gefahr eines unerwünschten Ablösens des Klebeverbands wird auf diese Weise weiter reduziert. Eine geeignete Trägerschicht mit daran angeordneter selbstklebender Haftschicht stellt beispielsweise das von der BSN medical GmbH unter der Bezeichnung "Fixomull® Stretch" vertriebe Produkt dar.

Die Trägerschicht ist bevorzugt ringförmig ausgebildet, so dass die Aussparung rahmenartig von der Trägerschicht umgeben ist.
Die Abstandseinrichtung ist vorteilhaft aus einem hygroskopen Material hergestellt, insbesondere aus einem Schaumstoff- oder vliesartig ausgebildeten Material. Entsprechend besitzt die Abstandseinrichtung die Fähigkeit, Feuchtigkeit aus der Umgebung anzuziehen und zu binden, was der Abfuhr von Wundsekret und anderen heilungshemmen-den flüssigen Substanzen zuträglich ist.

Gemäß einer Ausgestaltung der vorliegenden Erfindung sind die zur Umgebung freiliegenden Flächen der Abstandseinrichtung wasserdicht ausgebildet. Eine solche wasserdichte Ausbildung kann insbesondere durch Vorsehen einer wasserdichten Beschichtung realisiert werden. Eine solche Ausgestaltung der Abstandseinrichtung ist dahingehend von Vorteil, dass im bestimmungsgemäßen Zustand des Klebeverbandes Feuchtigkeit aus der Umgebung nicht durch die Abstandseinrichtung zur Wunde gelangen kann.

Die Abdeckschicht ist aus einem flexiblen Material hergestellt, insbesondere aus einem Gewebe- Entsprechend kann die Abdeckschicht Verformungen der Abstandseinrichtung problemlos folgen, wenn äußere Kräfte auf die Abstandseinrichtung einwirken.

Die Maschenweite der Abdeckschicht beträgt vorteilhaft 1,5 mm² oder weniger. Eine derart engmaschige Abdeckschicht ist dahingehend von Vorteil, dass Insekten, wie insbesondere Fliegen, nicht zur Wunde gelangen können. Auf diese Weise werden Infektionen sicher verhindert, die auf die Übertragung von Erregern durch Insekten oder eine Eiablage in der Wunde zurückzuführen sind.

Gemäß einer Ausgestaltung der vorliegenden Erfindung ist die Abdeckschicht wasserabweisend ausgebildet und/oder mit einem UV-Schutz versehen. Die wasserabweisende Ausbildung ist dahingehend von Vorteil, dass einem Verkleben der Abdeckschicht mit der Wunde, sollte die Abdeckschicht durch äußere Krafteinwirkung auf die Wunde gedrückt werden, entgegengewirkt wird. Das Vorsehen eines UV-Schutzes beschleunigt bekanntlich die Wundheilung. Ein UV-Schutz kann beispielsweise in Form einer entsprechenden Beschichtung realisiert werden. Alternativ oder zusätzlich kann die Abdeckschicht unterseitig, also auf der der zu versorgenden Wunde zugewandten Seite, eine Anti-Haftschicht aufweisen, beispielsweise in Form einer Aluminiumbeschichtung, die ein Verkleben der Wunde mit der Abdeckschicht verhindert.

Bevorzugt ist/sind die Abdeckschicht und/oder die Abstandseinrichtung lösbar an der Trägerschicht angeordnet, insbesondere unter Verwendung einer lösbaren Klettverschluss- oder Klebeverbindung. Eine solche lösbare Anordnung ermöglicht ein wiederholtes Entfernen und erneutes Anbringen der Abdeckschicht. Entsprechend ist die zu therapierende Wunde auf Wunsch stets frei zugänglich, um diese beispielsweise zu reinigen oder mit Arzneimitteln zu versorgen.

Vorteilhaft umfasst der Klebeverband eine die Haftschicht abdeckende und lösbar mit dieser verbundene Trennschutzschicht. Als Trennschutzschicht kann beispielsweise ein Plastikfilm dienen, der aus Polypropylen, Polyolefin oder dergleichen hergestellt ist. Die Trennschutzschicht schützt die Haftschicht vor äußeren Einwirkungen. Sie wird zur Freilegung der Haftschicht abgezogen, sobald der Klebeverband über einer Wunde fixiert werden soll.

Bevorzugt ist eine wasserabweisende Schutzschicht vorgesehen, die derart lösbar an dem Klebeverband befestigbar ist, dass sie zumindest die Abstandseinrichtung oberseitig abdeckt. Die Schutzschicht kommt dann zum Einsatz, wenn die zu therapierende Wunde beispielsweise bei starkem Regen vor eindringender Feuchtigkeit geschützt werden soll. Sie kann derart ausgebildet sein, dass sie lösbar auf der Abdeckschicht angeordnet werden kann. Alternativ kann die Ausgestaltung der Schutzschicht auch derart erfolgen, dass die Abdeckschicht wahlweise durch die Schutzschicht ersetzt werden kann. Hierzu wird die Schutzschicht entsprechend der Abdeckschicht mit einem Klettverschluss, einem Klebestreifen oder dergleichen versehen.

Ein bevorzugtes Anwendungsgebiet des erfindungsgemäßen Klebeverbands ist die Veterinärmedizin.

Weitere Vorteile und Merkmale der vorliegenden Erfindung werden anhand der nachfolgenden Beschreibung einer Ausführungsform des erfindungsgemäßen Klebeverbandes unter Bezugnahme auf die beiliegende Zeichnung deutlich. Darin ist
- Figur 1: eine schematische perspektivische Explosionsansicht eines Klebeverbandes gemäß einer ersten Ausführungsform der vorliegenden Erfindung;
- Figur 2: eine Seitenansicht des in Figur 1 dargestellten Klebeverbandes und
- Figur 3: eine schematische perspektivische Unteransicht einer Schutzschicht, die in Verbindung mit dem in den Figuren 1 und 2 dargestellten Klebeverband verwendet werden kann.

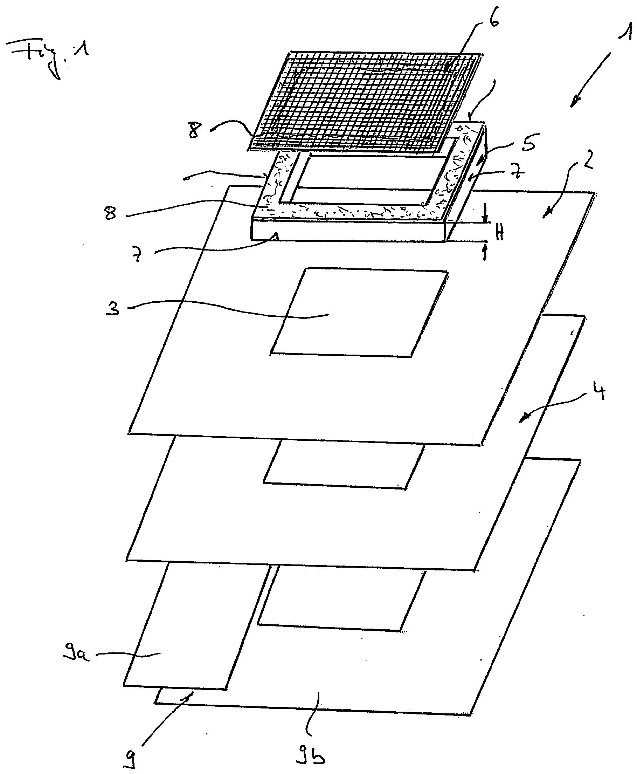

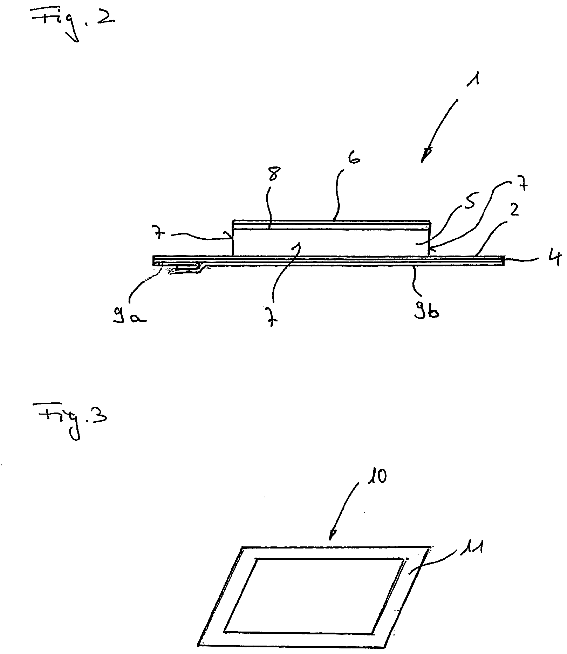

Die Figuren 1 und 2 zeigen einen Klebeverband 1 gemäß einer Ausführungsform der vorliegenden Erfindung, der zum Schutz und zur Therapierung offener Wunden dient. Der Klebeverband 1 umfasst eine flexible Trägerschicht 2, die eine die Wunde im bestimmungsgemäßen Zustand freiliegende Aussparung 3 definiert, eine an der Unterseite der Trägerschicht 2 vorgesehene selbstklebende Haftschicht 4, die im bestimmungsgemäßen Zustand zur Fixierung des Klebeverbands 1 am Körper eines Menschen oder eines Tieres dient, eine an der Oberseite der Trägerschicht 2 angeordnete, auswärts von dieser vorstehende und die Aussparung 3 rahmenartig umgebende Abstandseinrichtung 5 und eine die Abstandseinrichtung 5 zumindest oberseitig vollständig abdeckende, gitterartig ausgebildete Abdeckschicht 6.

Die Trägerschicht 2 ist aus einem stretchfähigen, eine Querelastizität aufweisenden Material hergestellt, wie beispielsweise aus einem stretchfähigen Gewebematerial oder Kunststoffmaterial in Form einer Kunststofffolie, eines Polyestervlies oder dergleichen. Entsprechend ist die Trägerschicht bei auftretender Zugbeanspruchung dehnfähig. Die Aussparung 3 ist im Wesentlichen mittig angeordnet, so dass sie von der Haftschicht 4 rahmenartig umrandet wird.

Bei der Haftschicht 4 handelt es sich um eine Selbstklebeschicht aus einem hautverträglichen Klebstoff. Bei dem Klebstoff kann es sich beispielsweise um einen Polyvinylether- oder einen Acryl-Klebstoff handeln, um nur einige Beispiele zu nennen. Die Haftschicht 4 ist vorliegend gleichmäßig über die gesamte Unterseite der Trägerschicht 2 aufgetragen, so dass die Form der Haftschicht 4 in Draufsicht im Wesentlichen der Form der Trägerschicht 2 in Draufsicht entspricht. Alternativ kann die Haftschicht 4 aber auch lediglich entlang des äußeren Randes der Trägerschicht 2 aufgetragen werden, solange ein ausreichender Halt des Klebeverbandes 1 im bestimmungsgemäßen Zustand gewährleistet ist.

Die an der Oberseite der Trägerschicht 2 befestigte Abstandseinrichtung 5 erstreckt sich entlang der Aussparung 3 der Trägerschicht 2, so dass die Aussparung 3 rahmenartig von der Abstandseinrichtung 5 eingefasst wird. Die Abstandseinrichtung 5 weist eine Höhe H auf, die bevorzugt im Bereich zwischen 3 und 15 mm liegt. Entsprechend steht die Abstandseinrichtung 5 aufwärts von der Trägerschicht 2 vor. Die Abstandseinrichtung 5 ist aus einem hygroskopen Material hergestellt, wie beispielsweise aus einem schaumstoff- oder vliesartig ausgebildeten Material. Somit besitzt die Abstandseinrichtung die Fähigkeit, Feuchtigkeit aus der Umgebung anzuziehen und zu binden. Die zur Umgebung freiliegenden Flächen 7 der Abstandseinrichtung 5 sind vorliegend wasserdicht ausgebildet. Dies kann beispielsweise durch Vorsehen einer wasserdichten Beschichtung realisiert werden. Die wasserdichte Ausbildung der Flächen 7 ist allerdings nicht zwingend erforderlich, so dass hierauf ggf. auch verzichtet werden kann. Die Abstandseinrichtung 5 ist vorliegend fest an der Oberseite der Trägerschicht 2 befestigt. Alternativ kann die Abstandseinrichtung 5 auch lösbar fixiert sein, beispielsweise unter Verwendung eines Klettverschlusses oder dergleichen. Ferner kann die Fixierung der Abstandseinrichtung beispielsweise auch zwischen der Trägerschicht 2 und der Haftschicht 4 erfolgen, auch wenn dies vorliegend nicht dargestellt ist.

Die Abdeckschicht 6 ist aus einem flexiblen Material hergestellt, insbesondere aus einem Gewebe. Entsprechend lässt sich die Abdeckschicht 6 unter Einwirkung äußerer Kräfte verformen. Die Abdeckschicht 6 ist lösbar über eine Klettverschlussverbindung 8 an der Oberseite der Abstandseinrichtung 5 gehalten und deckt diese vollständig ab. Somit kann die Abdeckschicht 6 wiederholt von der Abstandseinrichtung 5 entfernt und wieder an dieser befestigt werden. Anstelle einer Klettverschlussverbindung 8 ist grundsätzlich auch die Verwendung einer Klebeverbindung möglich, die sich wiederholt öffnen und schließen lässt. Die Maschenweite der Abdeckschicht 6 beträgt vorliegend 1,5 mm² oder weniger. Damit ist eine Luftzirkulation durch die Abdeckschicht 6 möglich, während das Eindringen von größeren Fremdkörpern und insbesondere das Eindringen von Insekten sicher verhindert wird.

Die Abdeckschicht 6 ist mit einer wasserabweisenden und einem UV-Schutz bildenden Beschichtung versehen. Unterseitig kann ferner eine Anti-Haftschicht vorgesehen sein, beispielsweise in Form einer Aluminiumbeschichtung, die ein Verkleben der Abdeckschicht 6 mit der Wunde verhindert, sollte die Abdeckschicht 6 durch von außen einwirkende Kräfte auf die Wunde gedrückt werden.

Der in den Figuren 1 und 2 dargestellte Klebeverband 1 umfasst ferner eine die Haftschicht abdeckende und lösbar mit dieser verbundene Trennschutzschicht 9 aus einem Plastikfilm, der aus Polypropylen, Polyolefin oder dergleichen hergestellt ist. Um die Entfernung der Trennschutzschicht 9 von der Haftschicht 4 zu erleichtern, ist die Trennschutzschicht 9 vorliegend aus zwei Trennschutzschichtelementen 9a und 9b gebildet, die einander teilweise überlappen, wobei das kleinere der beiden Trennschutzschichtelemente 9a U-förmig umgebogen ist. Grundsätzlich kann die Trennschutzschicht 9 natürlich auch durch ein einzelnes Trennschutzschichtelement realisiert werden, das die gesamte Haftschicht 4 abdeckt. Alternativ kann die Trennschutzschicht auch durch eine Verpackung gebildet werden, in welcher der Klebeverband 1 verpackt ist.

Zum Verbinden einer offenen Wunde mit dem in den Figuren 1 und 2 dargestellten Klebeverband 1 wird in einem ersten Schritt die Trennschutzschicht 9 entfernt, um die Haftschicht 4 freizulegen. Anschließend wird der Klebeverband derart oberhalb der Wunde positioniert, dass die Wunde vollständig in der Aussparung 3 in der Trägerschicht 2 aufgenommen ist. Daraufhin wird die Trägerschicht 2 auf der Haut/dem Fell des Patienten festgedrückt, wodurch der Klebeverband über die Haftschicht 4 fixiert wird. Dank der gitterartigen Ausbildung der Abdeckschicht 6 wird die Wunde stets mit ausreichend Frischluft versorgt, wodurch ein schneller Heilungsprozess gewährleistet wird. In Abhängigkeit von der Maschenweite der Abdeckschicht 6 kann die Wunde durch die Abdeckschicht 6 hindurch betrachtet werden, was die Beobachtung des Heilungsprozesses erleichtert. Die Abstandseinrichtung 5 stellt dabei sicher, dass die Wunde selbst nicht mit dem Klebeverband 1 in Berührung kommt, insbesondere nicht mit der Abdeckschicht 6. Zur Versorgung der Wunde können, wenn die Maschenweite der Abdeckschicht 6 hinreichend groß gewählt ist, Medikamente von außen durch die Abdeckschicht 6 auf die Wunde gesprüht werden. Auch ist ein Bestrahlen oder Lasern der Wunde durch die Abdeckschicht hindurch möglich. Alternativ kann dank der lösbaren Anordnung der Abdeckschicht 6 an der Abstandseinrichtung 5 die Abdeckschicht 6 temporär entfernt werden, so dass die Wunde zur Reinigung oder zur medikamentösen Behandlung frei zugänglich ist. Wundsekrete werden aufgrund der hygroskopen Ausbildung der Abstandseinrichtung 5 von dieser aufgesaugt, was ebenfalls dem Heilungsprozess zuträglich ist.

Werden auf die von dem Klebeverband 1 vorstehende Abstandseinrichtung 5 äußere Kräfte ausgeübt, wenn beispielsweise ein mit dem Klebeverband 1 behandeltes Tier den Klebeverband 1 über einen externen Gegenstand abstreifen will, so werden diese Kräfte dank der flexiblen Ausbildung der Abstandseinrichtung 5 durch eine entsprechende Ausweichbewegung der Abstandseinrichtung 5 absorbiert und/oder umgelenkt, so dass ein Abreißen der Abstandseinrichtung 5 sicher verhindert wird. Die flexible Ausbildung der Abdeckschicht 6 sorgt dafür, dass die Abdeckschicht 6 den Bewegungen der Abstandseinrichtung 5 stets folgen kann. Zugkräfte, die über die Abstandseinrichtung 5 auf die Trägerschicht 2 übertragen werden, führen aufgrund des stretchfähigen Materials lediglich zu einer Materialdehnung, nicht aber zu einem Abriss der Trägerschicht 2 vom Körper des Patienten.

Die Abstandseinrichtung 5 kann grundsätzlich auch lösbar an der Trägerschicht 2 angeordnet sein, was beispielsweise mit Hilfe einer Klettverschlussverbindung realisierbar ist. In diesem Fall ist die Abstandseinrichtung 5 austauschbar, was dann von Vorteil ist, wenn sich diese schon stark mit Wundsekret oder dergleichen vollgesaugt hat.

Figur 3 zeigt eine Schutzschicht 10, die aus einem wasserabweisenden Material hergestellt ist. Die Abmessungen der Schutzschicht 10 entsprechen im Wesentlichen den Abmessungen der Abdeckschicht 6, so dass letztere vollständig von der Schutzschicht 10 abgedeckt werden kann. Die Schutzschicht 10 ist an ihrer Unterseite mit einer Klebeverbindung 11 versehen, so dass sie lösbar ab der Abdeckschicht 6 befestigt werden kann. Die Schutzschicht 10 kann wahlweise zum Einsatz kommen, beispielsweise dann, wenn ein mit dem Klebeverband 1 behandeltes Tier bei starkem Regen auf der Weide steht und ein Eindringen von Regenwasser in den Klebeverband 1 verhindert werden soll. Bei nachlassendem Regen kann die Schutzschicht 10 dann wieder entfernt werden. An dieser Stelle sei darauf hingewiesen, dass die Schutzschicht 10 einen optionalen Bestandteil des Klebeverbandes 1 darstellt.

Der zuvor beschriebene Klebeverband 1 zeichnet sich insbesondere dadurch aus, dass er während der gesamten Therapiedauer grundsätzlich nicht entfernt werden muss. Eine Versorgung der Wunde ist entweder durch die Abdeckschicht 6 oder nach vorübergehender Entfernung der Abdeckschicht 6 auch ohne ein Abnehmen des Klebeverbandes 1 problemlos möglich. Dies spart zum einen natürlich Zeit. Zum anderen wird das Gefahrenpotential bei Tieren reduziert, die sich gegen einen zum Teil sehr schmerzhaften Verbandswechsel zur Wehr setzen. Ein weiterer wesentlicher Vorteil besteht darin, dass der Klebeverband 1 von einem Tier kaum abgestreift werden kann, da die beim Abstreifversuch erzeugten, von außen auf den Klebeverband 1 einwirkenden Kräfte lediglich zu einer ausweichenden Verformung der Abstandseinrichtung 5 und/oder der Abdeckschicht 6 und/oder der Trägerschicht 2 führen, weshalb die Kräfte im Regelfall nicht ausreichen, um den Klebeverband 1 vollständig zu lösen oder gar zu zerstören. Darüber hinaus lässt sich der Klebeverband 1 an jeder Stelle eines menschlichen oder tierischen Körpers problemlos und sicher positionieren, ohne bei der Fixierung des Klebeverbands 1 die Beweglichkeit einzuschränken. Ferner verhindert der Klebeverband 1 ein Durchfeuchten der Wunde. Die Luft kann zirkulieren, was einen Temperaturausgleich mit der Umgebung ebenso wie ein schnelles Heilen der Wunde fördert.

## Patentansprüche

1. Klebeverband (1) zum Schutz und zur Therapierung offener Wunden, umfassend
- eine flexible Trägerschicht (2), die eine die Wunde im bestimmungsgemäßen Zustand freilegende Aussparung (3) definiert,
- eine an der Unterseite der Trägerschicht (2) vorgesehene selbstklebende Haftschicht (4), die im bestimmungsgemäßen Zustand zur Fixierung des Klebeverbands (1) dient,
- eine auswärts von der Haftschicht (4) vorstehende und die Aussparung (3) rahmenartig umgebende Abstandseinrichtung (5) und
- eine die Abstandseinrichtung (5) zumindest oberseitig abdeckende, gitterartig ausgebildete Abdeckschicht (6), die aus einem flexiblen Material hergestellt ist,
wobei die Abstandseinrichtung (5) derart flexibel ausgebildet ist, dass sie sich unter Einwirkung äußerer Kräfte nachgiebig verformt, und die Trägerschicht (2) allseitig unter der Abstandseinrichtung (5) vorsteht.

2. Klebeverband (1) nach Anspruch 1, **dadurch gekennzeichnet, dass** die Trägerschicht (2) aus einem stretchfähigen Material hergestellt ist, insbesondere aus einem stretchfähigen Gewebe- oder Kunststoffmaterial.

3. Klebeverband (1) nach einem der vorhergehenden Ansprüche, **dadurch gekennzeichnet, dass** die Trägerschicht (2) ringförmig ausgebildet ist.

4. Klebeverband (1) nach einem der vorhergehenden Ansprüche, **dadurch gekennzeichnet, dass** die Abstandseinrichtung (5) aus einem hygroskopen Material hergestellt ist, insbesondere aus einem schaumstoff- oder vliesartig ausgebildeten Material.

5. Klebeverband (1) nach einem der vorhergehenden Ansprüche, **dadurch gekennzeichnet, dass** die zur Umgebung freiliegenden Flächen (7) der Abstandseinrichtung (5) wasserdicht ausgebildet sind, insbesondere mit einer wasserdichten Beschichtung versehen sind.

6. Klebeverband (1) nach einem der vorhergehenden Ansprüche, **dadurch gekennzeichnet, dass** die Höhe der Abstandseinrichtung (5) im Bereich von 3 mm bis 15 mm liegt.

7. Klebeverband (1) nach einem der vorhergehenden Ansprüche, **dadurch gekennzeichnet, dass** die Abdeckschicht (6) aus einem Gewebe hergestellt ist.

8. Klebeverband (1) nach einem der vorhergehenden Ansprüche, **dadurch gekennzeichnet, dass** die Maschenweite der Abdeckschicht (6) 1,5 mm² oder weniger beträgt.

9. Klebeverband (1) nach einem der vorhergehenden Ansprüche, **dadurch gekennzeichnet, dass** die Abdeckschicht (6) wasserabweisend ausgebildet und/oder mit einem UV-Schutz versehen und/oder unterseitig mit einer Anti-Haftschicht versehen ist.

10. Klebeverband (1) nach einem der vorhergehenden Ansprüche, **dadurch gekennzeichnet, dass** die Abdeckschicht (6) und/ oder die Abstandseinrichtung (5) lösbar an der Trägerschicht (2) angeordnet ist/ sind, insbesondere unter Verwendung einer lösbaren Klettverschluss- oder Klebeverbindung (8).

11. Klebeverband (1) nach einem der vorhergehenden Ansprüche, **dadurch gekennzeichnet, dass** eine die Haftschicht (4) abdeckende und lösbar mit dieser verbundene Trennschutzschicht (9) vorgesehen ist.

12. Klebeverband (1) nach einem der vorhergehenden Ansprüche, **dadurch gekennzeichnet, dass** eine wasserabweisende Schutzschicht vorgesehen ist, die derart lösbar an dem Klebeverband (1) befestigbar ist, dass sie zumindest die Abstandseinrichtung (5) oberseitig abdeckt.

13. Verwendung eines Klebeverbands (1) nach einem der vorhergehenden Ansprüche im Bereich der Veterinärmedizin.

## Claims

1. Adhesive dressing (1) for the protection and therapy of open wounds, comprising
- a flexible carrier layer (2) defining a recess (3) exposing the wound in the intended application,
- a self-adhesive adhesive layer (4) which is provided on the underside of the carrier layer (2) and which serves to fix the adhesive dressing (1) in the intended application,
- a spacer device (5) projecting outwards from the adhesive layer (4) and frame-like surrounding the recess (3) and
- a grid-like covering layer (6) which covers the spacer device (5) at least on the upper side and which is made of a flexible material,
wherein the spacer device (5) being flexible in such a way that it deforms yieldingly under the action of external forces, and the carrier layer (2) projecting on all sides under the spacer device (5).

2. Adhesive dressing (1) according to claim 1, **characterized in that** the carrier layer (2) is made of a stretchable material, in particular from a stretchable textile or plastic material.

3. Adhesive dressing (1) according to one of the preceding claims, **characterized in that** the carrier layer (2) is of annular design.

4. Adhesive dressing (1) according to one of the preceding claims, **characterized in that** the spacer device (5) is made of a hygroscopic material, in particular of a foamed material or of a non-woven material.

5. Adhesive dressing (1) according to one of the preceding claims, **characterized in that** the surfaces (7) of the spacer device (5) which are exposed to the environment are watertight, in particular are provided with a watertight coating.

6. Adhesive dressing (1) according to one of the preceding claims, **characterized in that** the height of the spacer device (5) is in the range from 3 mm to 15 mm.

7. Adhesive dressing (1) according to one of the preceding claims, **characterized in that** the covering layer (6) is made of a textile.

8. Adhesive dressing (1) according to one of the preceding claims, **characterized in that** the mesh size of the covering layer (6) is 1.5 mm² or less.

9. Adhesive dressing (1) according to one of the preceding claims, **characterized in that** the covering layer (6) is water-repellent and/or is provided with UV protection and/or is provided on the underside with an anti-adhesive layer.

10. Adhesive dressing (1) according to one of the preceding claims, **characterized in that** the covering layer (6) and/or the spacer device (5) is/are detachably arranged on the carrier layer (2), in particular using a detachable hook-and-loop fastener or adhesive connection (8).

11. Adhesive dressing (1) according to one of the preceding claims, **characterized in that** a separating protective layer (9) is provided covering the adhesive layer (4) and being detachably attached thereto.

12. Adhesive bandage (1) according to one of the preceding claims, **characterized in that** a water-repellent protective layer is provided which can be detachably fixed at the adhesive dressing (1) in such a way that it covers at least the spacer device (5) on the upper side.

13. Use of an adhesive dressing (1) according to one of the preceding claims in the field of veterinary medicine.

## Revendications

1. Pansement adhésif (1) pour protéger et traiter des plaies ouvertes, comprenant
- une couche de support flexible (2) définissant un évidement (3) exposant la plaie dans l'état prévu,
- une couche adhésive auto-adhésive (4) qui est prévue sur la face inférieure de la couche de support (2) et sert à fixer la bande adhésive (1) à l'état prévu,
- un dispositif d'espacement (5) faisant saillie vers l'extérieur depuis la couche adhésive (4) et en forme de cadre entourant l'évidement (3) et
- une couche de recouvrement (6) en forme de grille qui recouvre le dispositif d'espacement (5) au moins sur la face supérieure et qui est réalisée en un matériau flexible,
le dispositif d'espacement (5) étant conçu de manière flexible de telle sorte qu'il se déforme elastique sous l'action de forces externes, et la couche de support (2) faisant saillie de tous côtés sous le dispositif d'espacement (5).

2. Pansement adhésif (1) selon la revendication 1, **caractérisé en ce que** la couche de support (2) est fabriquée à partir d'un matériau extensible, en particulier d'un tissu extensible ou d'une matière plastique.

3. Pansement adhésif (1) selon l'une des revendications précédentes, **caractérisé en ce que** la couche de support (2) est annulaire.

4. Pansement adhésif (1) selon l'une des revendications précédentes, **caractérisé en ce que** le dispositif d'espacement (5) est constitué d'un matériau hygroscopique, en particulier d'un matériau en forme de mousse ou de nappe.

5. Pansement adhésif (1) selon l'une des revendications précédentes, **caractérisé en ce que** les surfaces (7) du dispositif d'espacement (5) qui sont exposées à l'environnement sont de construction étanche à l'eau, en particulier sont pourvues d'un revêtement étanche.

6. Pansement adhésif (1) selon l'une des revendications précédentes, **caractérisé en ce que** la hauteur du dispositif d'espacement (5) est comprise entre 3 mm et 15 mm.

7. Pansement adhésif (1) selon l'une des revendications précédentes, **caractérisé en ce que** la couche de recouvrement (6) est constituée d'un tissu.

8. Pansement adhésif (1) selon l'une des revendications précédentes, **caractérisé en ce que** le maillage de la couche de recouvrement (6) est de 1,5 mm² ou moins.

9. Pansement adhésif (1) selon l'une des revendications précédentes, **caractérisé en ce que** la couche de recouvrement (6) est hydrofuge et/ou munie d'une protection UV et/ou d'une couche anti-adhésive sur la face inférieure.

10. Pansement adhésif (1) selon l'une des revendications précédentes, **caractérisé en ce que** la couche de recouvrement (6) et/ou le dispositif d'espacement (5) sont disposés de manière détachable sur la couche de support (2), en particulier à l'aide d'une fermeture auto-agrippante ou d'une liaison adhésive (8) détachable.

11. Pansement adhésif (1) selon l'une des revendications précédentes, **caractérisé en ce qu'**il est prévu une couche de protection et de séparation (9) recouvrant la couche adhésive (4) et reliée de manière détachable à celle-ci.

12. Pansement adhésif (1) selon l'une des revendications précédentes, **caractérisé en ce qu'**il est prévu une couche de protection hydrofuge qui peut être fixée de manière détachable sur le pansement adhésif (1) de telle sorte qu'elle recouvre au moins le dispositif d'espacement (5) sur sa face supérieure.

13. Utilisation d'un pansement adhésif (1) selon l'une des revendications précédentes dans le domaine de la médecine vétérinaire.
